# EUROPEAN PATENT APPLICATION

(11) **EP 4 778 473 A1**
(43) Date of publication of application: **22.07.2026**
(21) Application number: 24865361.0
(22) Date of filing: 05.09.2024
(51) Int. Cl.: A61B 10/00, G16H 20/00

(54) **PROCESSING DEVICE, PROCESSING PROGRAM, PROCESSING METHOD, AND PROCESSING SYSTEM**

(30) Priority: 11.09.2023 JP 2023146721
(71) Applicant: National Cancer Center, Tokyo 104-0045 (JP)
(72) Inventor: OGAWA Asao, Tokyo 104-0045 (JP); TAKESHITA Nobuyoshi, Tokyo 104-0045 (JP)
(74) Representative: Reddie & Grose LLP
(86) International application number: PCT/JP2024/031927
(87) International publication number: WO 2025/057861

(57) **Abstract**

Provided are a processing device, a processing program, a processing method, and a processing system capable of more efficiently determining the onset of delirium.

Provided is a processing device including at least one processor, in which the at least one processor is configured to execute processing of acquiring subject information indicating a state of a subject who receives a medical act from a medical facility via a communication interface, acquiring a determination result of an onset of delirium by inputting the acquired subject information to a trained determination model used for determining the onset of delirium, and outputting the acquired determination result via the communication interface.

## Description

### Technical Field

The present disclosure relates to a processing device, a processing program, a processing method, and a processing system for determining the onset of delirium.

### Background Art

Conventionally, a system for managing a patient who is hospitalized in a medical facility has been known. For example, Patent Literature 1 discloses a "nursing support system comprising a server and a client, wherein the server includes a storage unit that records nursing data of a plurality of patients, a search processing unit that searches the storage unit for and acquires a plurality of pieces of nursing data of a patient whose progress table showing a state of the patient in time series is created by using a graph and a table, and a transmission unit that transmits the acquired nursing data, the client includes a reception unit that receives the nursing data transmitted from the transmission unit, and a progress table creation unit that creates the progress table of the patient on the basis of the received nursing data, and the search processing unit extracts the nursing data of an item necessary for creating the graph and the table in the progress table from among a plurality of items of the nursing data recorded in the storage unit".

### Citation List

### Patent Literature

Patent Literature 1: JP 2006-268613 A

### Summary of Invention

### Technical Problem

Therefore, based on the above-described technology, an object of the present disclosure is to provide a processing device, a processing program, a processing method, and a processing system capable of more efficiently determining the onset of delirium by various embodiments.

### Solution to Problem

According to one aspect of the present disclosure, there is provided a "processing device including at least one processor, in which the at least one processor is configured to execute processing of acquiring subject information indicating a state of a subject who receives a medical act from a medical facility via a communication interface, acquiring a determination result of an onset of delirium by inputting the acquired subject information to a trained determination model used for determining the onset of delirium, and outputting the acquired determination result via the communication interface".

According to one aspect of the present disclosure, there is provided a "processing program causing, in a computer including at least one processor, the at least one processor to function to acquire subject information indicating a state of a subject who receives a medical act from a medical facility via a communication interface, acquire a determination result of an onset of delirium by inputting the acquired subject information to a trained determination model used for determining the onset of delirium, and output the acquired determination result via the communication interface".

According to one aspect of the present disclosure, there is provided a "processing method executed by at least one processor in a computer including the at least one processor, the processing method including: acquiring subject information indicating a state of a subject who receives a medical act from a medical facility via a communication interface; acquiring a determination result of the onset of delirium by inputting the acquired subject information to a trained determination model used for determining onset of delirium; and outputting the acquired determination result via the communication interface".

According to one aspect of the present disclosure, there is provided a "processing system including the processing device described above, and a terminal device carried by a nurse who provides the medical act to the subject from the medical facility and configured to receive a determination result of the onset of delirium acquired by the processing device by being connected to the processing device via a communication network".

### Advantageous Effects of Invention

According to the present disclosure, it is possible to provide a processing device, a processing program, a processing method, and a processing system capable of more efficiently determining the onset of delirium.

Note that the above effects are merely exemplary for convenience of description, and are not restrictive. In addition to or instead of the above effects, any effect described in the present disclosure or an effect obvious to those skilled in the art can be exhibited.

### Brief Description of Drawings

Fig. 1 is a block diagram illustrating a configuration of a processing system 1 according to an embodiment of the present disclosure.
Fig. 2 is a block diagram illustrating a configuration of a processing device 100 according to the embodiment of the present disclosure.
Fig. 3A is a diagram conceptually illustrating a subject management table stored in an information providing device 400 according to the embodiment of the present disclosure.
Fig. 3B is a diagram conceptually illustrating a cause management table stored in the information providing device 400 according to the embodiment of the present disclosure.
Fig. 4 is a diagram illustrating a processing flow executed in the processing system 1 according to the embodiment of the present disclosure.
Fig. 5 is a diagram illustrating a processing flow executed in a model generation device 300 according to the embodiment of the present disclosure.
Fig. 6 is a diagram illustrating a processing flow executed in the model generation device 300 according to the embodiment of the present disclosure.
Fig. 7 is a diagram illustrating an example of a screen output from the processing device 100 according to the embodiment of the present disclosure.
Fig. 8 is a diagram illustrating an example of a screen output from the processing device 100 according to the embodiment of the present disclosure.
Fig. 9 is a diagram illustrating an example of a screen output from the processing device 100 according to the embodiment of the present disclosure.
Fig. 10 is a diagram illustrating an example of a screen output from the processing device 100 according to the embodiment of the present disclosure.

### Description of Embodiments

### 1. Outline of Processing System 1

A processing system 1 according to the present disclosure is used to acquire subject information indicating a state of a subject who receives a medical act in a medical facility and output a determination result of the onset of delirium. In particular, the processing system 1 is used to determine a risk of the onset of delirium in the future for a subject or the like who is hospitalized in a medical facility, and further to determine the degree of the onset cause.

Here, delirium is one of consciousness disorder mainly caused by physical abnormalities such as pain, dehydration, constipation, and fever, or by use of drugs. Delirium is a condition presenting various mental symptoms such as cognitive dysfunction, hallucinatory delusions, and mood swings. Such delirium can be classified into three types: a hyperactive type in which a highly active and psychomotor excited state such as restlessness occurs, a hypoactive type in which a decrease in activity due to cloudiness of consciousness such as drowsiness occurs, and a mixed type thereof. When delirium is developed, not only the influence on the treatment by a medical worker but also the influence on a subject himself/herself and family members around the subject is extremely large, such as causing an accident such as tumbling or falling or accompanying secondary complications. In particular, it is said that 10% to 30% of general hospitalized patients, about 40% of hospitalized patients due to elderly progressive cancer for a purpose other than treatment, and 40% or more of hospitalized patients to a palliative care unit develop delirium, and the increase in medical cost due to the prolonged hospitalization and the mental burden on patients and their families are also large.

As described above, it is extremely beneficial to determine a risk of the onset of delirium and further determine the degree of a cause of the onset of delirium. Therefore, the processing system 1 can particularly determine a risk of the onset of delirium and determine the degree of a cause of the onset of delirium on the basis of subject information indicating a state of a subject.

Note that, in the present disclosure, the "subject" may be any person as long as the person is a subject who receives a medical act in a medical facility, and is not limited to only a person having a specific attribute. Therefore, such a subject includes all humans such as a patient, an examinee, a diagnosis subject, a healthy person, and a person requiring care. In particular, as described above, the onset of delirium is frequently observed in hospitalized patients, and thus the hospitalized patients are suitably exemplified.

In the present disclosure, the "user" may be any person who directly or indirectly uses the processing device 100 in the processing system 1, and is not limited to only a person having a specific attribute. Therefore, such a user includes all persons such as a medical worker, a subject himself/herself, and a close relative such as a family member who supports the subject. In particular, as described above, symptoms of delirium are often observed in hospitalized patients, and a nurse, a care worker, a close relative such as a family member of the subject, or the like is suitably exemplified from the viewpoint of daily providing a medical act or long-term care to such hospitalized patients.

Furthermore, in the present disclosure, the "medical worker" may be any person as long as the person is a person engaged in a medical act, and is not limited to only a person having a specific attribute. Therefore, such a medical worker includes all persons such as a doctor, a nurse, a medical technician, a pharmacist, a medical clerical worker, a medical institution staff and a manager, an emergency worker, a person in charge of a medical device and medical equipment, a caregiver, a nursing home staff and a manager, and a worker of an institution that provides medical acts such as chiropractic, bone setting, and massage. In particular, as described above, symptoms of delirium are often observed in hospitalized patients, and a nurse is preferably exemplified from the viewpoint of daily providing a medical act to such hospitalized patients.

Furthermore, in the present disclosure, the "medical act" is not limited to a medical practice provided on the basis of medicine for treatment, diagnosis, or prevention of a human disease, and includes any of pseudo medical practices such as chiropractic, bone setting, massage, or nursing care. In addition, similarly, the "medical facility" may be any facility as long as the medical acts are provided, and includes not only a medical institution in which the medical practices as described above are provided but also a period in which the pseudo medical practices as described above are provided.

In addition, in the present disclosure, terms such as "determination" are used, but these do not necessarily mean a definite determination by a doctor. For example, these terms naturally include that a user other than a doctor uses the processing system 1 such that a determination is made by the processing device 100. The term "determination" also includes assisting a doctor or the like in making a definite determination or diagnosis.

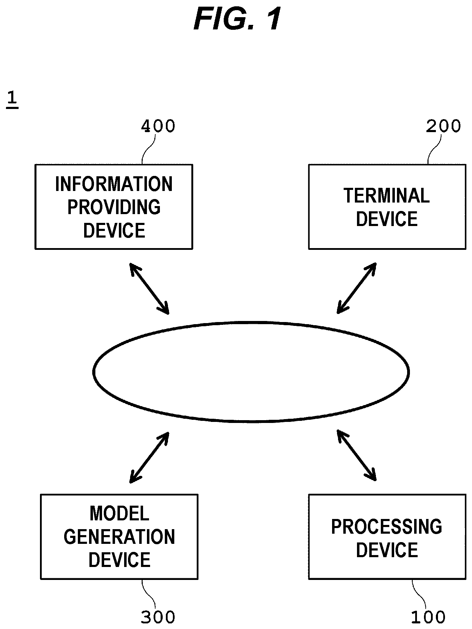

Fig. 1 is a block diagram illustrating a configuration of the processing system 1 according to an embodiment of the present disclosure. According to Fig. 1, the processing system 1 includes at least a processing device 100 configured to determine the onset of delirium and a terminal device 200 configured to acquire the determination result of the onset of delirium from the processing device 100. Furthermore, the processing system 1 may include an information providing device 400 configured to provide the processing device 100 with subject information indicating a state of a subject, and a model generation device 300 for generating a trained determination model used when the processing device 100 determines the onset of delirium. The processing device 100, the terminal device 200, the model generation device 300, and the information providing device 400 are communicatively connected to each other via at least one of a wireless network and a wired network.

### 2. Configuration of Processing Device 100

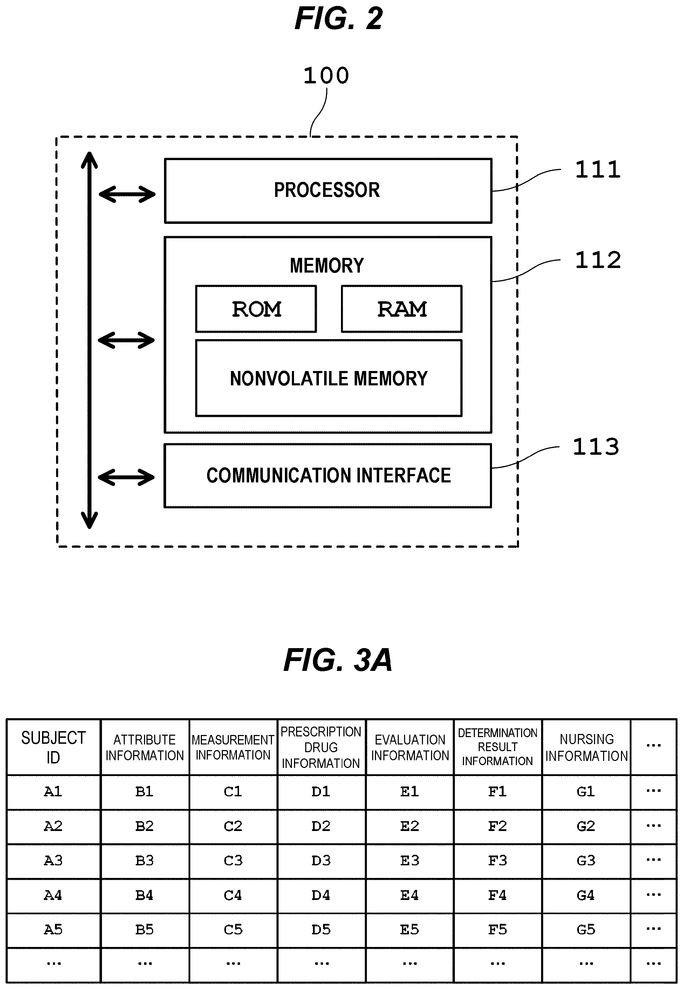

Fig. 2 is a block diagram illustrating a configuration of the processing device 100 according to the embodiment of the present disclosure. According to Fig. 2, the processing device 100 includes a processor 111, a memory 112, and a communication interface 113. These constituents are electrically connected to each other via a control line and a data line. Note that the processing device 100 does not need to include all of the constituents illustrated in Fig. 2, and can be configured by omitting some of the constituents, or another constituent can be added. Typically, a server device, a database device, a laptop PC device, a desktop PC device, a smartphone device, or a tablet device is used as the processing device 100, but a cloud server device is preferably used from the viewpoint of service provision efficiency or an on-premises server device is preferably used from the viewpoint of security. In addition, in the processing device 100, it is not necessary for an integrated processing device to perform all of the processes, and it is also possible for a plurality of processing devices to perform the processes in a distributed manner. In such a case, the processing device 100 includes a plurality of processing devices that distributes the processes.

In the processing device 100, the processor 111 functions as a control unit that controls the processing device 100 or other constituents of the processing system 1 on the basis of programs stored in the memory 112. Specifically, the processor 111 executes a "process of acquiring subject information indicating a state of a subject who receives a medical act from a medical facility via the communication interface 113", a "process of acquiring a determination result of the onset of delirium by inputting the acquired subject information to a trained determination model used for determining the onset of delirium", a "process of outputting the acquired determination result via the communication interface 113", and the like on the basis of the programs stored in the memory 112. The processor 111 is mainly configured by one or a plurality of CPUs, but a GPU, an FPGA, or the like may be appropriately combined.

The memory 112 includes a RAM, a ROM, a nonvolatile memory, an HDD, an SSD, and the like, and functions as a storage unit. The memory 112 stores instruction commands for various types of control of the processing system 1 according to the present embodiment as programs. Specifically, the memory 112 stores programs to be executed by the processor 111, such as a "process of acquiring subject information indicating a state of a subject who receives a medical act from a medical facility via the communication interface 113", a "process of acquiring a determination result of the onset of delirium by inputting the acquired subject information to a trained determination model used for determining the onset of delirium", and a "process of outputting the acquired determination result via the communication interface 113". In addition to the programs, the memory 112 mainly stores various types of information stored in a subject management table, a cause management table, and the like received from the information providing device 400 in accordance with the progress of processing.

The communication interface 113 functions as a notification unit for transmitting and receiving various types of information to and from the information providing device 400 and the terminal device 200 connected via a wired or wireless network. Examples of the communication interface 113 include various devices such as a connector for wired communication such as a USB and an SCSI, a transmission/reception device for broadband wireless communication such as a wireless LAN, Bluetooth (registered trademark), and LTE, and wireless communication such as infrared rays, and various connection terminals for a printed mounting board and a flexible mounting board. The communication interface 113 receives subject information from the information providing device 400 or transmits a determination result to the terminal device 200, for example.

Note that a configuration of the terminal device 200 will not be specifically mentioned, but the terminal device 200 includes, for example, a processor, a memory, a communication interface, an input interface, and an output interface. The terminal device 200 receives an instruction input by the user via the input interface, and transmits various requests to the processing device 100 via the communication interface. Furthermore, the terminal device 200 receives various types of information such as a determination result acquired by the processing device 100 via the communication interface, and outputs the information (displays the information on a display or prints the information by using a printer) via the output interface. Examples of the terminal device 200 include a laptop personal computer, a desktop personal computer, a smartphone, and a tablet terminal. As described above, the terminal device 200 is mainly used by a nurse, a caregiver, a close relative such as a family member of the subject, or the like from the viewpoint of the symptoms of delirium being frequently observed in hospitalized patients and providing a daily medical act or long-term care to the hospitalized patients. In the present disclosure, the processing device 100 and the terminal device 200 are distinguished from each other, but of course, they can function as the processing device 100 integrally. In such a case, the processing device 100 includes an input interface and an output interface.

Although the configuration of the model generation device 300 for generating a trained determination model will not be specifically described, the model generation device 300 includes, for example, a processor, a memory, an input interface, an output interface, and a communication interface. As such a device, a laptop personal computer, a desktop personal computer, a smartphone, a tablet terminal, a server device, or a combination thereof is used. Note that, in the present disclosure, the processing device 100 and the model generation device 300 are distinguished from each other, but of course, they can function as the processing device 100 integrally.

Although a specific configuration of the information providing device 400 will not be particularly described, the information providing device 400 includes, for example, a processor, a memory, and a communication interface, and includes an input interface, an output interface, and the like as necessary. Examples of such an information providing device 400 include a measurement device itself, a device for controlling the measurement device, an interview device, an electronic medical record device, a database device or a server device in which information input thereto is recorded, or a combination thereof. The information providing device 400 reads necessary information in response to a request received from the processing device 100, for example, and provides the necessary information to the processing device 100. Note that, in the present disclosure, the processing device 100 and the information providing device 400 are distinguished from each other, but of course, they can function as the processing device 100 integrally.

### 3. Information Stored in The Information Providing Device 400

Fig. 3A is a diagram conceptually illustrating a subject management table stored in the information providing device 400 according to the embodiment of the present disclosure. The information stored in the subject management table is read from the information providing device 400 according to the progress of the processing of the processor 111 of the processing device 100, and is updated as needed and stored in the information providing device 400.

According to Fig. 3A, the subject management table stores attribute information, measurement information, prescription drug information, evaluation information, determination result information, nursing information, and the like in association with subject ID information. The "subject ID information" is information for specifying each subject with information unique to each subject. The subject ID information is generated every time a new subject is registered by the user via the terminal device 200. The "attribute information" is information regarding an attribute of each subject and is one of the subject information indicating a state of the subject. Examples of the attribute information include subject name information, date of birth information, gender information, address information, height information, weight information, and interview information. Note that each piece of information exemplified here is merely an example of the attribute information, and any information indicating information of a subject can be used. The attribute information is acquired by being input through the input interface by any user in the terminal device 200.

The "measurement information" is information measured by the measurement device and is one of the subject information indicating the state of the subject. Examples of the measurement information include various types of information such as a body temperature measured by a thermometer, a heart rate measured by a heart rate meter (the maximum value and the minimum value), a blood pressure measured by a sphygmomanometer (the maximum value, the minimum value, and a range thereof), an oxygen saturation measured by a pulse oximeter (the maximum value, the minimum value, and a range thereof), a pulse measured by a pulsometer, respective numerical values of white blood cells, red blood cells, hemoglobin, platelets, and hematrics, and the like measured by a blood inspection device, respective numerical values of a pH, white blood cells, nitrites, bilirubin, ketone bodies, and the like measured by a urine analysis device, echo data measured by an ultrasonic inspection device, electrocardiogram data measured by an electrocardiogram inspection device, and CT data measured by a CT device. Among these, the respective numerical values of body temperature, heart rate, blood pressure, oxygen saturation, pulse, white blood cells, red blood cells, hemoglobin, platelets, hematrics, and the like are preferably used as those useful for determining the onset of delirium. Note that each piece of information exemplified here is merely an example of the measurement information, and any information indicating the information of the subject can be used. The measurement information is acquired by being received from the measurement device via a communication interface, being input by any user via an input interface, or the like.

The "prescription drug information" is information regarding a drug prescribed to a subject or a drug (including a medical narcotic) administered for treatment, and is one of the subject information indicating the condition of the subject. The prescription drug information may include a prescription amount, a dosage method, and a dosage frequency in addition to a name of an actually prescribed drug. In addition, the prescription drug information may include not only information regarding a drug prescribed this time but also information regarding a drug prescribed in the past. The prescription drug information is acquired by being input via an input interface by any user in the terminal device 200.

The "evaluation information" is information indicating a result of evaluation made on delirium or a disease associated with delirium for a subject and is one of the subject information indicating the condition of the subject. Examples of the evaluation information include the presence or absence of dementia, the presence or absence of a brain matrix disease, the presence or absence of heavy drinking of alcohol, and a history of delirium. The evaluation information is acquired by being input via an input interface by any user in the terminal device 200. Note that the evaluation information exemplified here is merely an example, and does not need to include all of them, and only some thereof may be used, or may be replaced with or added to other evaluation information.

The "determination result information" is information indicating a result of determining the onset of delirium. The determination result information includes risk determination information indicating a result of determining the risk of the onset of delirium and cause determination information indicating a result of determining the degree of the cause of the onset of delirium. The risk determination information may be any of a numerical value indicating the degree of risk of the onset of delirium, a classification indicating the degree of "high", "medium", "low", "level 1", "level 2", "level 3", or the like. In the cause determination information, a possibility that, for each of "drug", "pain", "dehydration", "constipation", and "fever" which are the causes of the onset of delirium, this will cause delirium, is indicated by a numerical value or a classification indicating the degree such as "high", "medium", "low", "level 1", "level 2", or "level 3". Note that these causes are examples, and may be information for specifying other symptoms. The determination result information is obtained by being processed by the processing device 100. In addition, the determination result information includes determination date information indicating the date on which each of the determination of the risk of the onset of delirium and the degree of the cause of the onset of delirium is determined, as necessary.

The "nursing information" is information indicating a history of a medical act provided in a medical facility. The medical act is mainly provided by a nurse and thus is considered as nursing information, but may naturally include information provided by a person other than a nurse. As the nursing information, different information is stored for each cause of the onset of delirium. In the case of the "drug", examples of the nursing information include:
· whether or not a drug that can cause delirium is used and a dose of the drug;
· whether or not a medical narcotic is used and a dose of the medical narcotic; and
· a check result of consistency between the dose and a disease that causes hospitalization. Furthermore, in the case of "pain", examples of the nursing information include:
   a check result of the presence or absence of pain; and
   an evaluation result of pain (a strength, a property, a site, etc.). Furthermore, in the case of "dehydration", examples of the nursing information include:
· whether or not to perform a blood test;
·a check result of vital information such as a body temperature and a heartbeat;
· a result of visual inspection; and
· a drinking situation and an amount thereof, and a proposal for a drinking method. In addition, in the case of "constipation", examples of the nursing information include:
   · the elapsed time from the last defecation;
   a state of the feces;
   the severity of the constipation based on an interview or a state of the intestines; and
   · whether or not to take a laxative and the amount of the laxative. In addition, in the case of "fever", examples of the nursing information include:
      · whether or not to perform a blood test;
      · a check result of vital information such as a body temperature and a heartbeat; and
      · whether or not to perform treatment such as taking an antipyretic, cooling, and moisturizing. The nursing information is acquired, for example, by being input via an input interface by any user in the terminal device 200 every time a nurse provides these medical acts on the basis of the determination result information.

The information stored in the subject management table may be stored in the information providing device 400, or may be stored in another processing device including the processing device 100, a database device, or the like.

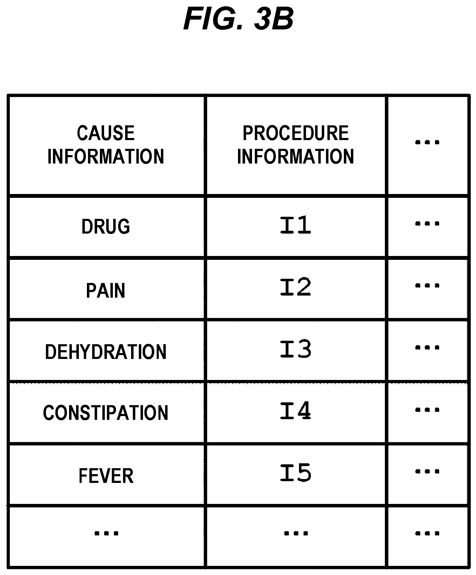

Fig. 3B is a diagram conceptually illustrating a cause management table stored in the information providing device 400 according to the embodiment of the present disclosure. The information stored in the cause management table is read from the information providing device 400 according to the progress of the processing of the processor 111 of the processing device 100, and is updated as needed and stored in the information providing device 400.

According to Fig. 3B, in the cause management table, procedure information and the like are stored in association with cause information for specifying each cause. Examples of the "cause information" include information specifying "drug", "pain", "dehydration", "constipation", and "fever" indicating the causes of the onset of delirium. That is, the information is information provided to correspond to each cause specified as the cause determination information in the determination result information. Note that the cause information exemplified here is merely an example, and it is not necessary to include all of them, and only a part thereof may be used, or may be replaced with or added to other cause information.

In the "procedure information", different information is stored for each cause specified by the cause determination information. The procedure information is information for proposing a medical act to be provided to the subject. That is, in a case where the cause information is a "drug", an example of the procedure information is information for making a proposal to:
· check whether or not a drug that may cause delirium is used and a dose of the drug;
· check whether or not a medical narcotic is used and a dose of the medical narcotic;
· check consistency between the dose and a disease that causes hospitalization; and
· check with a doctor in charge. In addition, in a case where the cause information is "pain", an example of the procedure information is information for making a proposal to:
   check the presence or absence of pain;
   evaluate pain (a strength, a property, a site, etc.); and
   · make consultation of a response level. In addition, in a case where the cause information is "dehydration", an example of the procedure information is information for making a proposal to:
      · check whether or not a blood test has been performed;
      · check vital information such as a body temperature and a heartbeat;
      to perform visual inspection; and
      check a water drinking status and an amount thereof and propose a water drinking method. In addition, in a case where the cause information is "constipation", an example of the procedure information is information for making a proposal to:
         · check the elapsed time from the last defecation;
         · check a state of feces;
         determine the severity of constipation based on the interview and the state of the intestines; and
         · determine whether or not a laxative has been taken and the amount of the laxative. In addition, in a case where the cause information is "fever", an example of the procedure information is information for making a proposal to:
            · check whether or not a blood test has been performed;
            · check vital information such as a body temperature and a heart rate;
            · and check treatment such as taking an antipyretic, cooling, and moisturizing.

Note that these pieces of procedure information are merely examples, and as a matter of course, information other than these may be included. In addition, in Fig. 3B, the procedure information is stored according to each symptom, but the procedure information may be stored according to not only the cause but also the accuracy of the determination of the cause. That is, in such a case, it is possible to select an optimal procedure according to not only the cause but also the accuracy.

The information stored in the cause management table may be stored in the information providing device 400, or may be stored in another processing device including the processing device 100, a database device, or the like.

### 4. Processing Flow Executed by Processing Device 100

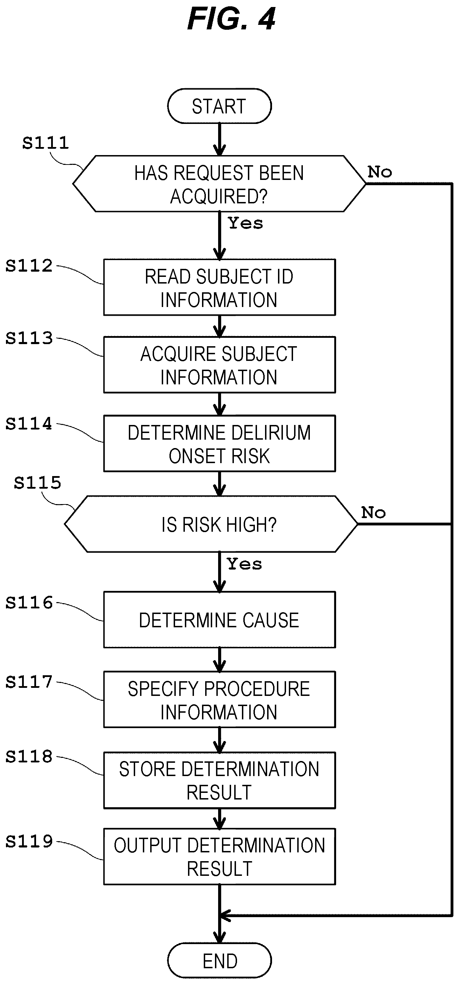

Fig. 4 is a diagram illustrating a processing flow executed in the processing device 100 according to the embodiment of the present disclosure. Specifically, Fig. 4 is a diagram illustrating a processing flow executed by the processor 111 of the processing device 100 by receiving, via the communication interface 113 of the processing device 100, a determination request that is transmitted by receiving an instruction input from a user via the input interface in the terminal device 200. The processing flow is mainly performed by the processor 111 of the processing device 100 reading and executing a program stored in the memory 112.

First, although not particularly illustrated, the processing flow is started by receiving, via the communication interface 113, a determination request transmitted by receiving an instruction input by the user via the input interface in the terminal device 200 (S111).

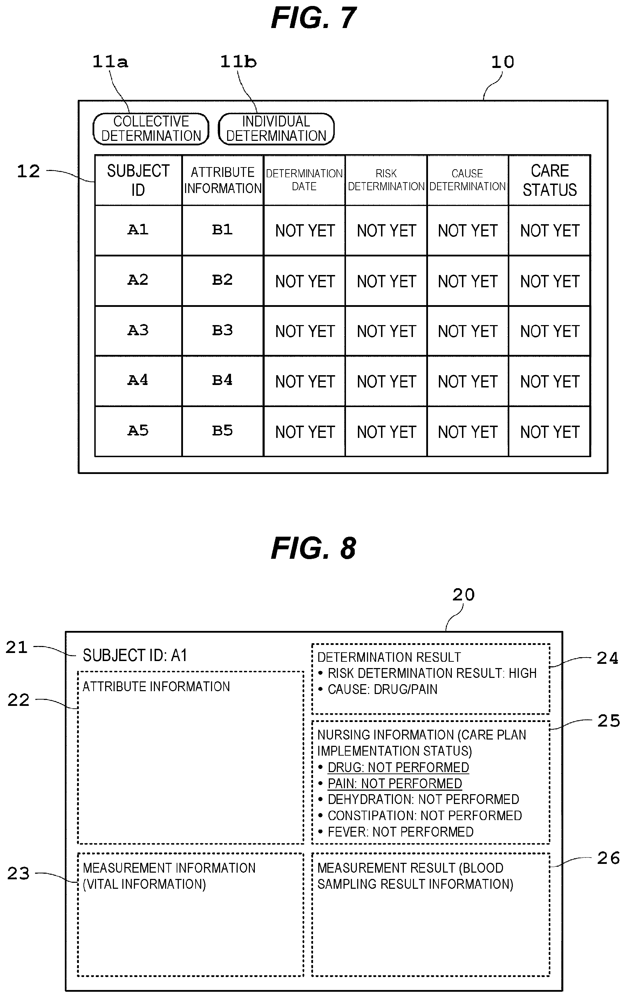

Here, Fig. 7 is a diagram illustrating an example of a screen output from the processing device 100 according to the embodiment of the present disclosure. Specifically, Fig. 7 is a diagram illustrating an example of a subject list screen 10 output to the display via the output interface in the terminal device 200. According to Fig. 7, the subject list screen 10 includes a collective determination icon 11a, an individual determination icon 11b, and a subject list region 12. The collective determination icon 11a is an icon for collectively making a request for determination of the onset of delirium for all subjects listed in the subject list region 12 or a plurality of subjects selected by receiving an instruction input from the user via the input interface. That is, when the instruction input for the collective determination icon 11a is received via the input interface, the processor of the terminal device 200 transmits, to the processing device 100 via the communication interface, the determination request including the subject ID information associated with all the subjects or the selected plurality of subjects and the request for the determination.

The individual determination icon 11b is an icon for making a request for the determination of the onset of delirium for one subject selected by receiving the instruction input by the user via the input interface. That is, when the instruction input for the individual determination icon 11b is received via the input interface, the processor of the terminal device 200 transmits, to the processing device 100 via the communication interface, a determination request including the subject ID information associated with one subject selected in advance and the request for determination.

In addition, the subject list region 12 displays a list of subjects acquired in advance from the subject management table. That is, the subject list region 12 includes attribute information, determination date information, risk determination information, cause determination information, and care status information in association with subject ID information of the subject. Among them, the attribute information is information read from the attribute information which is one of the subject information stored in the subject management table. The determination date information is information read from the determination result information stored in the subject management table. Specifically, the determination date information indicates the date on which the determination of the risk of the onset of delirium and the determination of the cause of the onset of delirium are made. Note that, as a matter of course, the expression form of the date may be any form, and may include up to time information. The risk determination information is information stored in the subject management table and read from the risk determination information indicating the result of the determination of the risk of the onset of delirium. Specifically, the risk determination information includes numerical values, classifications, and the like indicating the degree of risk obtained as a result of the determination. The cause determination information is information stored in the subject management table and read from the cause determination information indicating a result of determining the degree of the cause of the onset of delirium. Specifically, the cause determination information includes the cause of the onset of delirium and, if necessary, information indicating the degree of accuracy of the cause. The care status information is information stored in the subject management table and read from the nursing information. Specifically, the care status information includes information indicating a history and progress of whether or not a medical act is provided in a medical facility on the basis of the nursing information.

In the subject list region 12, each piece of the above information is displayed as a list in association with the subject ID information. The processor of the terminal device 200 can select the subject by receiving the instruction input of the user with respect to the row of the desired subject via the input interface. Although not specifically illustrated, the processor enables discrimination between the selected subject and the unselected subject by changing the display mode of the row corresponding to the selected subject (for example, by changing the display mode such that the row is to be highlighted). When the instruction input for the collective determination icon 11a or the individual determination icon 11b is received as described above, the subject ID information of the selected subject is transmitted to the processing device 100 as a determination subject.

Note that, in Fig. 7, "not yet" is displayed for all of the determination date information, the risk determination information, the cause determination information, and the care status information in the subject list region 12. This indicates that, as a result of referring to the corresponding information in the subject management table, the processing related to the determination has not yet been performed, and the determination result information, the nursing information, and the like have not yet been stored.

Referring to Fig. 4 again, as described above, upon receiving the determination request via the communication interface 113, the processor 111 of the processing device 100 reads the subject ID information included in the determination request (S112). The processor 111 transmits the read subject ID information to the information providing device 400 via the communication interface 113.

Here, upon receiving the subject ID information from the processing device 100, the processor of the information providing device 400 refers to the subject management table and reads subject information such as attribute information, measurement information, prescription drug information, and evaluation information associated with the received subject ID information. The processor of the information providing device 400 transmits the read subject information to the processing device 100 via the communication interface.

The processor 111 of the processing device 100 receives and acquires the subject information transmitted via the communication interface 113 (S113). Note that, although the case of receiving the subject information from the remotely installed information providing device 400 has been described here, the information providing device 400 and the processing device 100 may be integrally configured. In this case, the subject information is acquired by being read from the subject management table stored in the memory 112 of the processing device 100.

Upon acquiring the subject information, the processor 111 determines the risk of the onset of delirium on the basis of the acquired subject information (S114). As an example, the determination is performed by using a trained risk determination model for determining the risk of the onset of delirium.

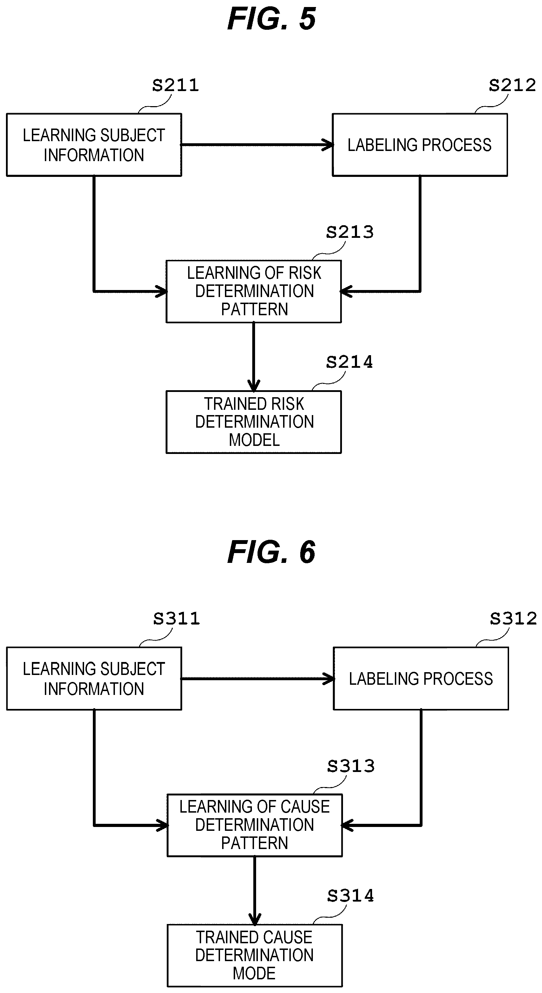

Here, Fig. 5 is a diagram illustrating a processing flow executed in the model generation device 300 according to the embodiment of the present disclosure. Specifically, Fig. 5 is a diagram illustrating a processing flow related to generation of the trained risk determination model used in the determination process in S114 in Fig. 4. The processing flow is mainly performed by the processor of the model generation device 300 reading and executing a program stored in the memory.

According to Fig. 5, the processor of the model generation device 300 acquires, for example, the subject information including the attribute information, the measurement information, the prescription drug information, and the evaluation information acquired in advance for each subject as learning subject information from the information providing device 400 (S211). Note that not only the subject information acquired from the actual subject as described above but also subject information generated through simulation can be similarly used as the learning subject information.

Next, the processor of the model generation device 300 executes processing of labeling with risk determination information indicating the risk of the onset of delirium on the basis of each piece of the acquired learning subject information (S212). The labeling is performed by a medical worker such as a doctor diagnosing whether or not a subject corresponding thereto develops delirium on the basis of each piece of learning subject information or assigning a numerical value or a classification indicating the degree of risk of the onset of delirium.

When the learning subject information and the risk determination information associated with the learning subject information are obtained, the processor performs machine learning of the determination pattern by using the learning subject information and the risk determination information (S213). As an example, the machine learning is performed by giving the learning subject information to a neural network configured by combining neurons and repeating learning while adjusting parameters of the respective neurons so that an output of the neural network becomes the same as the risk determination information. The processor acquires the trained risk determination model (for example, the neural network and the parameters) (S214). As a result, the processor ends the generation of the trained risk determination model. Note that the acquired trained risk determination model may be transmitted to the processing device 100 via the communication interface and stored in the memory 112 of the processing device 100. Alternatively, the trained risk determination model may be stored in a memory of the model generation device 300, another processing device, a server device, or the like.

Referring to Fig. 4 again, the processor 111 acquires risk determination information indicating the risk of the onset of delirium as an output by inputting the subject information to the trained risk determination model generated as described above. As described above, it is possible to efficiently determine the risk of the onset of delirium by using the trained risk determination model. Note that, here, the risk determination information is acquired by using the trained risk determination model, but may be acquired by using another method as a matter of course. For example, the processor 111 may prepare a risk management table in which the subject information and the risk determination information are associated in advance, and acquire the risk determination information by referring to the risk management table on the basis of the acquired subject information.

Next, the processor 111 refers to the acquired risk determination information to determine whether or not the result of risk determination is "high" indicating that the risk of the onset of delirium is high (S115). When determining that the result is "high", the processor 111 further determines the degree of the cause of the onset of delirium on the basis of the subject information of the subject for which the result is "high" (S116). That is, the determination indicates the risk of the occurrence of each symptom for each cause of the onset of delirium. As an example, the determination is performed by using a trained cause determination model for determining the cause of the onset of delirium.

Here, Fig. 6 is a diagram illustrating a processing flow executed in the model generation device 300 according to the embodiment of the present disclosure. Specifically, Fig. 6 is a diagram illustrating a processing flow related to generation of the trained cause determination model used in the determination process in S116 in Fig. 4. The processing flow is mainly performed by the processor of the model generation device 300 reading and executing a program stored in the memory. In the example in Fig. 6, such a trained cause determination model is generated in advance for each factor that causes the onset of delirium (drug, pain, dehydration, constipation, and fever), and first, a case where the factor that causes delirium is a "drug" will be described.

According to Fig. 6, the processor of the model generation device 300 acquires, for example, the subject information including the attribute information, the measurement information, the prescription drug information, and the evaluation information acquired in advance for each subject as the learning subject information from the information providing device 400 (S311). Note that not only the subject information acquired from the actual subject as described above but also subject information generated through simulation can be similarly used as the learning subject information.

Next, the processor of the model generation device 300 executes processing of labeling with cause determination information indicating whether or not delirium has been developed due to the "drug" on the basis of each piece of the acquired learning subject information (S312). The labeling is performed by a medical worker such as a doctor determining, for a subject who has developed delirium, whether the cause thereof is a drug on the basis of each piece of learning subject information.

When each piece of learning subject information and the cause determination information associated with the learning subject information are obtained, the processor performs machine learning of the determination pattern by using the learning subject information and the cause determination information (S313). As an example, the machine learning is performed by giving the learning subject information to a neural network configured by combining neurons and repeating learning while adjusting parameters of the respective neurons so that an output of the neural network becomes the same as the cause determination information. The processor acquires the trained cause determination model (for example, the neural network and the parameters) for determining whether the drug is a cause (S314).

In addition, the processor performs labeling for causes (pain, dehydration, constipation, and fever) other than the drug through similar processing, and acquires each trained cause determination model for determining whether or not each of pain, dehydration, constipation, and fever is a cause. As a result, the processor ends the generation of each trained cause determination model.

Note that each of the acquired trained cause determination models may be transmitted to the processing device 100 via the communication interface and stored in the memory 112 of the processing device 100, or may be stored in a memory of the model generation device 300, another processing device, a server device, or the like.

In addition, in the example in Fig. 6, the case of generating and acquiring the trained cause determination model for each cause has been described. However, the present disclosure is not limited thereto, and the presence or absence of a plurality of causes may be determined by one trained cause determination model. Such a trained cause determination model is generated by a medical worker labeling which one of a drug, pain, dehydration, constipation, and fever is the cause on the basis of each piece of learning subject information for a subject who has developed delirium. The learning subject information and the cause are input to the model generation device 300, and machine learning of the determination pattern is executed, so that one trained cause determination model capable of determining each cause is acquired. As described above, by using one trained cause determination model, it is possible to reduce a processing load related to generation of the trained cause determination model.

Returning to Fig. 4 again, the processor 111 acquires cause determination information indicating the degree of the cause of the onset of delirium as an output by inputting the subject information to the trained cause determination model generated as described above. As described above, it is possible to efficiently identify the cause of the onset of delirium by using the trained cause determination model. Note that, here, the cause determination information is acquired by using the trained cause determination model, but may be acquired by using another method as a matter of course. For example, the processor 111 may prepare a cause determination table in which the subject information and the cause determination information are associated in advance, and acquire the cause determination information by referring to the cause determination table on the basis of the acquired subject information.

Next, the processor 111 refers to the cause management table on the basis of the acquired cause determination information, and specifies procedure information for proposing improvement of the cause (S117). Specifically, the processor 111 specifies the cause for which the determination of "high" indicating that the possibility of causing the onset of delirium is high is made in the cause determination information. The processor 111 specifies and reads the procedure information associated with the specified cause.

The determination result of the risk of the onset of delirium (risk determination information) acquired in S114 in Fig. 4, the determination result of the degree of each cause of the onset of delirium (that is, the possibility of mainly causing the onset of delirium) acquired in S116 in Fig. 4, and the procedure information (cause determination information) for the determination result are transmitted to the information providing device 400 via the communication interface 113 together with the subject ID information. The processor of the information providing device 400 that has received the information stores the information in the subject management table in association with the subject ID information (S118).

In addition, the processor 111 transmits determination result information including the determination result of the risk of the onset of delirium (risk determination information) acquired in S114 in Fig. 4, the determination result of the degree of each cause of the onset of delirium (that is, the possibility of mainly causing the onset of delirium) acquired in S116 in Fig. 4, and procedure information (cause determination information) for the determination result to the terminal device 200 that has transmitted the determination request via the communication interface 113 (S119).

Here, Fig. 8 is a diagram illustrating an example of a screen output from the processing device 100 according to the embodiment of the present disclosure. Specifically, Fig. 8 is a diagram illustrating an example of a determination result check screen 20 output to the display via the output interface in the terminal device 200. The determination result check screen 20 is displayed, for example, when the processor of the terminal device 200 receives an instruction input by the user to select a row of a desired subject via the input interface in the subject list screen 10 illustrated in Fig. 7.

The determination result check screen 20 includes a subject ID region 21 including subject ID information for specifying a subject, an attribute region 22 including attribute information of the subject, a measurement region 23 and a measurement region 26 including measurement information of the subject, a determination result region 24 including determination result information of the subject, and a nursing region 25 including nursing information of the subject. That is, the attribute information, which is one of the subject information stored in the subject management table, is read and displayed in the attribute region 22. In the measurement region 23, vital information that is a numerical value of a body temperature, a heartbeat, or the like is read and displayed among the pieces of measurement information that is one of the subject information stored in the subject management table. In addition, in the determination result region 24, risk determination information indicating the determination result of the risk of the onset of delirium and cause determination information indicating the determination result of the degree of each cause of the onset of delirium are displayed among the pieces of determination result information output from the processing device 100. Furthermore, in the nursing region 25, information indicating whether or not a medical act for improving each cause is provided is displayed on the basis of the nursing information stored in the subject management table. In the measurement region 26, each numerical value obtained by the blood test of white blood cells, red blood cells, and the like among the pieces of measurement information which is one of the subject information stored in the subject management table is read and displayed.

Here, in the nursing region 25, information indicating whether or not a medical act for improving each cause is provided is displayed as described above, and in the example in Fig. 8, "not performed" is displayed in any case. Among them, upon receiving an instruction input from the user via the input interface for the positions corresponding to the "drug" and the "pain" determined to have a high possibility of causing the onset of delirium, the processor of the terminal device 200 outputs a proposal screen for proposing the procedure information among the pieces of determination result information output from the processing device 100 to the medical worker or the like.

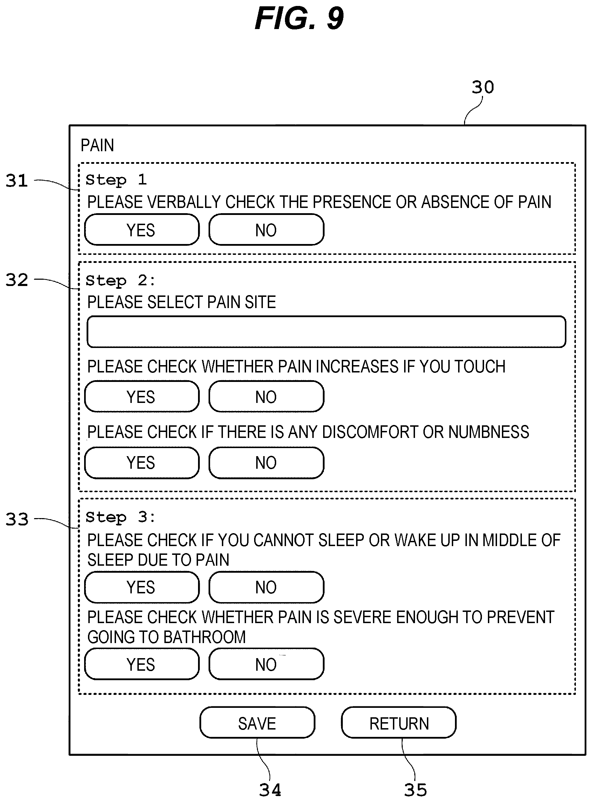

Fig. 9 is a diagram illustrating an example of a screen output from the processing device 100 according to the embodiment of the present disclosure. Specifically, Fig. 9 is a diagram illustrating an example of a proposal screen 30 output to the display via the output interface in the terminal device 200. The proposal screen 30 is displayed, for example, when the processor of the terminal device 200 receives an instruction input of the user via the input interface for a desired cause in the nursing region 25 of the determination result check screen 20 as described above.

According to Fig. 9, out of the "drug" and the "pain" determined to have a high possibility of causing the onset of delirium, procedure information for the "pain" is included. Specifically, the proposal screen 30 includes a proposal region 31 to a proposal region 33 for proposing a medical act to be provided to the subject to a medical worker such as a nurse in each step and inputting a result of the medical act. First, the proposal region 31 includes an indication of "Please verbally check the presence or absence of pain" and an icon for selecting a check result in order to propose the subject to check the presence or absence of pain. In addition, the proposal region 32 includes indications of "Please select the pain site", "Please check whether the pain increases if you touch", and "Please check if there is any discomfort or numbness.", and an input box and an icon for inputting and selecting each check result, in order to propose that the subject should check the site and property of the pain in a case where there is the pain. Furthermore, the proposal region 33 includes indications of "Please check if you cannot sleep or wake up in the middle of sleep due to pain." and "Please check whether pain is severe enough to prevent going to a bathroom" and icons for selecting respective check results in order to propose that the subject check the intensity of pain. That is, the proposal screen 30 includes an indication based on the procedure information of the cause management table.

Note that, although the pain has been described as an example in Fig. 9, different procedure information is displayed depending on each cause. The procedure information to be displayed is generated to correspond to the procedure information of the cause management table. Furthermore, in the example in Fig. 9, the case where the three proposal regions of the proposal region 31 to the proposal region 33 are included has been described, but the proposal regions may be provided to correspond to the procedure information, and the number thereof may be one, two, or four or more depending on the procedure information.

Here, the proposal screen 30 includes a save icon 34 and a return icon 35 at a lower portion thereof. Upon receiving the instruction input of the user for the save icon 34 via the input interface, the processor of the terminal device 200 saves each piece of information currently input to each of the proposal region 31 to the proposal region 33, and returns to the subject list screen. Specifically, the processor transmits each piece of information input in each of the proposal region 31 to the proposal region 33 to the information providing device 400 via the communication interface in order to store the information in the nursing information of the subject management table. Upon receiving an information update request from the terminal device, the processor 111 of the processing device 100 reads the nursing information and the like stored in the subject management table from the information providing device 400 and transmits the nursing information and the like to the terminal device 200 via the communication interface. Upon receiving the latest subject information via the communication interface, the processor of the terminal device 200 performs control to display the information on the subject list screen. Upon receiving an instruction input of the user for the return icon 35, the processor performs control to display the subject list screen without performing the above processing.

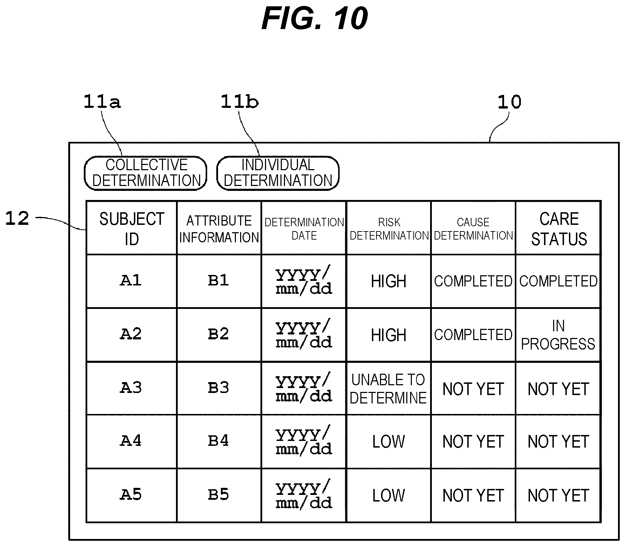

Fig. 10 is a diagram illustrating an example of a screen output from the processing device 100 according to the embodiment of the present disclosure. Specifically, Fig. 10 is a diagram illustrating an example of the subject list screen 10 output to the display via the output interface in the terminal device 200. The subject list screen 10 is displayed, for example, when the processor of the terminal device 200 receives an instruction input for the save icon 34 via the input interface in the proposal screen 30 as described above.

According to Fig. 10, the configuration of the screen is similar to that of the example illustrated in Fig. 7. In Fig. 10, as described in Figs. 8 and 9, a state is illustrated in which determination of the risk of the onset of delirium and determination of the degree of the cause thereof are performed by the processing of S114 and S116 in Fig. 4, and provision of a medical act is being performed. Therefore, when attention is paid to the subject whose subject ID information is "A1", the determination date information, the risk determination information, the cause determination information, and the care status information are indicated as "not yet" in Fig. 7. Specifically, the determination date, the result of the risk determination, the indication indicating that the cause has been determined, and the indication indicating that the provision of the medical act has been completed are displayed.

Referring to Fig. 4 again, by outputting the determination result information in S119, the terminal device 200 can perform various displays according to the determination result information. In particular, in Fig. 8, by displaying the determination result information output by the processing device 100 as the determination result information on the terminal device 200, a user including a medical worker such as a nurse can more quickly ascertain the determination result and the cause of the onset of delirium. Furthermore, in Fig. 9, by displaying the procedure information output by the processing device 100 on the terminal device 200, a medical worker such as a nurse can more reliably and quickly provide the subject with a necessary medical act. In addition, in Fig. 10, by displaying the latest status of the determination result information or the like in a list form, it is possible to quickly ascertain the latest status.

Thus, the processing flow is ended.

In Fig. 4, the trained risk determination model is used in S114, and the trained cause determination model is used in S116 to determine the risk of the onset of delirium and the degree of the cause of the onset of delirium, respectively. However, the determination result may be acquired by using only one of the trained determination models.

In addition, the degree of the cause of the onset of delirium is determined in S116 only for the subject whose risk of the onset of delirium is determined to be "high" in S114. However, the degree of the cause of the onset of delirium may be determined for all subjects regardless of the degree of the risk of the onset of delirium.

In addition, only for the cause for which the possibility of causing delirium is determined to be "high" in S117, the procedure information corresponding thereto is specified. As a result, it is possible to specify a cause to be more preferentially addressed and to preferentially perform improvement thereof. However, the present disclosure is not limited thereto, and procedure information may be specified for all causes and output to the terminal device 200. In this case, indications corresponding to all pieces of procedure information are displayed on the screens displayed in Figs. 8 and 9.

As described above, in the present embodiment, it is possible to provide a processing device, a processing program, a processing method, and a processing system capable of more efficiently determining the onset of delirium.

### 9. Others

In the above embodiment, each piece of information illustrated in Fig. 3A has been described as an example of the learning subject information and the subject information used for each determination. However, these are merely examples, and for example, an MRI device, an X-ray device, an electrocardiograph device, an ultrasonic diagnostic device, an endoscope device, a sphygmomanometer device, other medical record information, other interview information, and the like can also be used in combination.

In the above embodiment, a case where a medical act is mainly provided by a nurse has been described. However, these are merely examples, and a medical act may be provided by, for example, a doctor, a medical technician, a pharmacist, a medical clerical worker, a medical institution staff and a manager, an emergency worker, a person in charge of a medical device and medical equipment, a caregiver, a nursing home staff and a manager, a worker of an institution that provides medical acts such as chiropractic, bone setting, and massage, and a close relative such as a family member who supports a subject. In such a case, the terminal device 200 may be available to each person exemplified above other than a nurse.

In the above embodiment, the degree of the cause of the onset of delirium is determined only when the acquired result of risk determination is "high" indicating that the risk of the onset of delirium is high (S115) (S116). However, instead of this, even when the risk of the onset is "low", the degree of the cause of the onset of delirium may be determined. That is, the determination in S115 may be omitted, and the degree of the cause of the onset of delirium in S116 may be determined in all cases.

The trained determination model described in the above embodiment is generated by using a neural network or a convolutional neural network. However, the present disclosure is not limited to these, and the trained determination model may be generated by using machine learning such as a nearest-neighbor method, a decision tree, a regression tree, and a random forest.

The processing and procedures described in the present specification can be realized not only by those explicitly described in the embodiments but also by software, hardware, or a combination thereof. Specifically, the processing and procedures described in the present specification are realized by mounting logic corresponding to the processes on a medium such as an integrated circuit, a volatile memory, a nonvolatile memory, a magnetic disk, or an optical storage. In addition, the processing and procedures described in the present specification can be implemented as a computer program and executed by various computers including a processing device and a server device.

Even if it is described that the processing and procedures described in the present specification are executed by a single device, a single piece of software, a single component, or a single module, such processing or procedures can be executed by a plurality of devices, a plurality of pieces of software, a plurality of components, and/or a plurality of modules. Furthermore, even if it is described that various types of information described in the present specification are stored in a single memory or storage unit, such information can be stored in a distributed manner in a plurality of memories provided in a single device or a plurality of memories disposed in a distributed manner in a plurality of devices. Furthermore, the software and hardware elements described in the present specification can be realized by integrating them into fewer constituents or decomposing them into more constituents.

### Reference Signs List

- 1: Processing system
- 100: Processing device
- 200: Terminal device
- 300: Model generation device
- 400: Information providing device

## Claims

1. A processing device comprising at least one processor, wherein the at least one processor is configured to execute processing of:
acquiring subject information indicating a state of a subject who receives a medical act from a medical facility via a communication interface;
acquiring a determination result of an onset of delirium by inputting the acquired subject information to a trained determination model used for determining the onset of delirium; and
outputting the acquired determination result via the communication interface.

2. The processing device according to claim 1, wherein the trained determination model is a trained risk determination model used for determining a risk of the onset of delirium.

3. The processing device according to claim 1, wherein the determination result is a result of determining a risk of the onset of delirium.

4. The processing device according to claim 1, wherein the trained determination model is a trained cause determination model used to determine a degree of a cause of the onset of the delirium.

5. The processing device according to claim 1, wherein the determination result is a result of determining a degree of a cause of the onset of the delirium.

6. The processing device according to claim 1, wherein the determination result is a result of determining a risk of an onset of delirium by inputting the acquired subject information to a trained risk determination model used for determining the risk of the onset of delirium, and determining a degree of a cause of the onset of delirium by inputting the subject information to a trained cause determination model used for determining the degree of the cause of the onset of delirium in a case where the risk of the onset of delirium is determined to be high.

7. The processing device according to claim 1, wherein the at least one processor selects proposal information of a medical act to be provided to the subject according to a result of determining a degree of a cause of the onset of the delirium.

8. The processing device according to claim 1, wherein the subject information is information acquired in a case where the subject is hospitalized in the medical facility.

9. The processing device according to claim 1, wherein the subject information includes at least one of attribute information indicating an attribute of the subject, measurement information generated by measuring a body of the subject, prescription drug information prescribed by a medical worker for the subject, and evaluation information indicating an evaluation related to delirium performed for the subject.

10. The processing device according to claim 1, wherein the determination result is output to a terminal device that provides the medical act to the subject in the medical facility or is available to a user who is a close relative of the subject.

11. A processing program for causing, in a computer including at least one processor, the at least one processor to function to:
acquire subject information indicating a state of a subject who receives a medical act from a medical facility via a communication interface;
acquire a determination result of an onset of delirium by inputting the acquired subject information to a trained determination model used for determining the onset of delirium; and
output the acquired determination result via the communication interface.

12. A processing method executed by at least one processor in a computer including the at least one processor, the processing method comprising:
a step of acquiring subject information indicating a state of a subject who receives a medical act from a medical facility via a communication interface;
a step of acquiring a determination result of an onset of delirium by inputting the acquired subject information to a trained determination model used for determining the onset of delirium; and
a step of outputting the acquired determination result via the communication interface.

13. A processing system comprising:
the processing device according to claim 1; and
a terminal device carried by a nurse who provides the medical act to the subject from the medical facility and configured to receive a determination result of the onset of delirium acquired by the processing device by being connected to the processing device via a communication network.
